# EUROPEAN PATENT SPECIFICATION

(11) **EP 2 311 813 B1**
(45) Date of publication and mention of the grant of the patent: **11.09.2013**
(21) Application number: 09771967.8
(22) Date of filing: 30.06.2009
(51) Int. Cl.: C07D 223/26

(54) **A METHOD FOR PREPARING 5H-CYANO-IMIDO STILBENE**
VERFAHREN ZUR HERSTELLUNG VON 5H-CYANOIMIDOSTILBEN
PROCÉDÉ DE PRÉPARATION DE 5H-CYANO-IMIDO STILBÈNE

(30) Priority: 04.07.2008 CN 200810062838
(43) Date of publication of application: 20.04.2011
(73) Proprietor: Zhejiang University Of Technology, Zhejiang 310014 (CN); Zhejiang Jiuzhou Pharmaceutical Co., Ltd., Jiaojiang District Taizhou City Zhejiang 318000 (CN)
(72) Inventor: SU, Weike, Zhejiang 310014 (CN); SHI, Xiangjun, Hangzhou 310014, P.R. (CN); CHE, Daqing, BRANTFORD, Ontario N3R 7M7 / CA (CA); LIN, Jinrong, Zhejiang 318000 (CN); HU, Kai, Zhejiang 318000 (CN)
(74) Representative: Rhein, Alain
(86) International application number: PCT/CN2009/072528
(87) International publication number: WO 2010/000197

(56) References cited:
- WO-A1-2008/012837
- CN-A- 101 328 146
- US-A- 4 436 660
- AHMAD, S.: "A Convenient Synthesis of Carbamazepine", INDIAN JOURNAL OF CHEMISTRY, vol. 27B, 1988, page 583, XP009158488,
- GÉRARDIN, ANDRÉ ET AL.: 'GLC Determination ofCarbamazepin Suitable for Pharmacokinetic Studies.' JOURNAL OF PHARMACEUTICAL SCIENCES vol. 64, no. 12, December 1975, pages 1940 - 1942, XP008141656
- SARAIE, TAKAHIRO ET AL.: 'A new Synthesis of Nitriles.' TETRAHEDRON LETT. vol. 14, no. 23, 1973, pages 2121 - 2124, XP025683671
- WEI, WENG LONG ET AL.: 'Composition and Application of Triphosgene.' JOURNAL OF TAIYUAN UNIVERSITY OF TECHNOLOGY vol. 32, no. 3, May 2001, pages 266 - 270, XP008141696
- SAHU: 'Devi Prasad Bis(trichloromethyl)carbonate/triethylamine : A convenient reagent for dehydration of aldoximes and carboxamides to nitriles.' INDIAN JOURNAL OF CHEMISTRY vol. 32B, no. 3, March 1993, pages 385 - 386, XP008141697
- LV, FENG ET AL.: 'Application of Triphosgene in Organic Synthesis.' INFORMATION RECORDING MATERIALS vol. 5, no. 3, 2004, pages 21 - 25, XP008141761

## Description

The present invention relates to a method for preparing 5-cyano-5H-dibenz[b,f]azepine.

5-cyano-5H-dibenz[b,f]azepine is an important intermediate of the new antiepileptic drug oxcarbazepine.

WO2008012837 discloses two methods for preparing 5-cyano-5H-dibenz[b,f]azepine, in one method, imino stilbene is taken as raw material, which reacts with cyaniding reagent (BrCN) to obtain 5-cyano-5H-dibenz[b,f]azepine; in the other method, carbamazepine is taken as raw material, which reacts with dehydrating reagent (P₂O₅ or POCl₃ or SOCl₂) to obtain 5-cyano-5H-dibenz[b,f]azepine.

The two methods of the above patent, one with highly toxic cyaniding reagent, and the other with phosphide or sulfide as dehydrating reagent, have large security risks and waste management problems, and do not meet the requirements of green chemistry.

The technical problem the present invention to solve is to provide a green method for preparing 5-cyano-5H-dibenz[b,f]azepine with simple and reasonable process, higher yield of the product and no three wastes.

In order to solve the above technical problem, the invention adopts the following technical scheme:
A method for preparing 5-cyano-5H-dibenz[b,f]azepine of formula (I) includes the following steps: carbamazepine of formula (II) and bis(trichloromethyl) carbonate of formula (III) are taken as raw materials which react in an organic solvent for 1∼30 hours at 20∼150°C, and reaction solution is post-treated to obtain the 5-cyano-5H-dibenz[b,f]azepine (I). The reaction equation is shown as below:

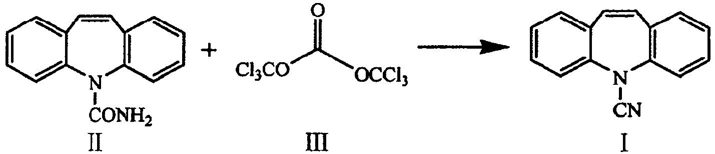

The said organic solvent of the present invention is selected from the group consisting of: benzene, toluene, xylene, chlorobenzene, dichloromethane, chloroform, carbon tetrachloride, dichloroethane, tetrahydrofuran, ethyl acetate, acetone and cyclohexane. Preferably, the said organic solvent is selected from the group consisting of: toluene, xylene, dichloromethane, chloroform, tetrahydrofuran, ethyl acetate, and acetone.

The molar ratio of carbamazepine to bis(trichloromethyl) carbonate is generally 1.0:0.2∼1.2, preferably 1.0:0.3∼0.8. In the preparation process, carbamazepine is added directly into the reactor containing the organic solvent. Bis(trichloromethyl) carbonate can be added directly into the reaction system, and also can be added into the reaction system in the form of liquid which is obtained by dissolving bis(trichloromethyl) carbonate with organic solvent first. The latter is recommended, so that the speed of adding bis(trichloromethyl) carbonate can be better controlled.

The amount of the said organic solvent in mass is recommended 5∼20 times of carbamazepine, preferably 5∼10 times.

Further, the reaction temperature is preferably 20∼80°C, the reaction time is preferably 4∼15 hours.

The post-treatment of the present invention may take the following steps: the reaction solution is washed with water, and then organic phase and water phase are separated. The organic phase is dried over anhydrous sodium sulfate, and then vacuum distillated to recycle solvent. The resulting residue is recrystallized with isopropanol to obtain 5-cyano-5H-dibenz[b,f]azepine (I).

The specifically recommended preparation is performed according to the following steps: an organic solvent and carbamazepine (II) are added into the reaction vessel. Then a solution consisting of bis(trichloromethyl) carbonate (III) in the same organic solvent is slowly dropped under vigorous stirring at room temperature. Following the addition, the reaction mixture is maintained at 20∼80°C for 4∼15 hours. After the reaction is complete, reaction solution is washed twice with water, and organic phase and water phase are separated. The organic phase is dried over anhydrous sodium sulfate and then vacuum distillated to recycle solvent. The resulting residue is recrystallized with isopropanol to obtain 5-cyano-5H-dibenz[b,f]azepine (I). The organic solvent is toluene, xylene, dichloromethane, chloroform, tetrahydrofuran, ethyl acetate or acetone. The molar ratio of carbamazepine to bis(trichloromethyl) carbonate is 1.0:0.3∼0.8, and the total amount of the organic solvent is 5∼10 times of the mass of carbamazepine.

Compared with the prior art, the main advantages of the present invention are in:

The process uses bis(trichloromethyl) carbonate to replace P₂O₅ or POCl₃ or SOCl₂ as a dehydrating reagent to prepare the 5-cyano-5H-dibenz[b,f]azepine, thereby greatly reducing the safety and environment protection hidden trouble at the source, meanwhile, the method has simple and reasonable process, and high yield of the product and is suitable for industrial production.

### Mode of the Invention

Thereafter the present invention will be discribed in detail combining the specific examples, however the scope of the invention are not limited by the examples.

### Example 1

The feed ratio of carbamazepine : bis(trichloromethyl) carbonate was 1:0.2 (molar ratio), the organic solvent was toluene, and the reaction temperature was the reflux temperature of toluene.

A 500mL four-necks flask equipped with a mechanical agitator, constant pressure funnel, reflux condenser and thermometer was charged with 23.60g carbamazepine and 100mL toluene. Open the agitator, and then a solution consisting of 5.94g bis(trichloromethyl) carbonate in 50mL toluene was added dropwise under vigorous stirring at room temperature. Following the addition, the reaction mixture was heated to reflux and then maintained for 5 hours. After that, the solution was stood to cool, washed twice with 100mL water, and then organic phase and water phase were separated. The organic phase was dried over anhydrous sodium sulfate, and then vacuum distillated to recycle solvent. The resulting residue was recrystallized with isopropanol to obtain 5-cyano-5H-dibenz[b,f]azepine. The yield of the product was 38.7%, and the purity was 91%.

### Example 2

The feed ratio of carbamazepine : bis(trichloromethyl) carbonate was 1:0.3 (molar ratio), the organic solvent was toluene, and the reaction temperature was the reflux temperature of toluene.

The other operations were the same as example 1, and 5-cyano-5H-dibenz[b,f]azepine was obtained. The purity of the product was 96%, and the yield was 80%.

### Example 3

The feed ratio of carbamazepine : bis(trichloromethyl) carbonate was 1:0.4 (molar ratio), the organic solvent was toluene, and the reaction temperature was the reflux temperature of toluene.

The other operations were the same as example 1, and 5-cyano-5H-dibenz[b,f]azepine was obtained. The purity of the product was 98.5%, and the yield was 96%.

### Example 4

The feed ratio of carbamazepine : bis(trichloromethyl) carbonate was 1:0.5 (molar ratio), the organic solvent was toluene, and the reaction temperature was the reflux temperature of toluene.

The other operations were the same as example 1, and 5-cyano-5H-dibenz[b,f]azepine was obtained. The purity of the product was 97%, and the yield was 95.7%.

### Example 5

The feed ratio of carbamazepine : bis(trichloromethyl) carbonate was 1:0.6 (molar ratio), the organic solvent was toluene, and the reaction temperature was the reflux temperature of toluene.

The other operations were the same as example 1, and 5-cyano-5H-dibenz[b,f]azepine was obtained. The purity of the product was 96%, and the yield was 95.8%.

### Example 6

The feed ratio of carbamazepine : bis(trichloromethyl) carbonate was 1:0.9 (molar ratio), the organic solvent was toluene, and the reaction temperature was the reflux temperature of toluene.

The other operations were the same as example 1, and 5-cyano-5H-dibenz[b,f]azepine was obtained. The purity of the product was 92%, and the yield was 95.8%.

### Example 7

The feed ratio of carbamazepine : bis(trichloromethyl) carbonate was 1:1 (molar ratio), the organic solvent was toluene, and the reaction temperature was the reflux temperature of toluene.

The other operations were the same as example 1, and 5-cyano-5H-dibenz[b,f]azepine was obtained. The purity of the product was 92%, and the yield was 95.6%.

### Example 8

The feed ratio of carbamazepine : bis(trichloromethyl) carbonate was 1:1.2 (molar ratio), the organic solvent was toluene, and the reaction temperature was the reflux temperature of toluene.

The other operations were the same as example 1, and 5-cyano-5H-dibenz[b,f]azepine was obtained. The purity of the product was 90%, and the yield was 94.8%.

### Example 9

The feed ratio of carbamazepine : bis(trichloromethyl) carbonate was 1:0.4 (molar ratio), the organic solvent was toluene, and the reaction temperature was 20°C.

A 500mL four-necks flask equipped with a mechanical agitator, constant pressure funnel, reflux condenser and thermometer was charged with 23.60g carbamazepine and 100mL toluene. Opened the agitator, and then a solution consisting of 11.88g bis(trichloromethyl) carbonate in 100mL toluene was added dropwise under vigorous stirring at room temperature. Following the addition, the reaction mixture was maintained at 20°C for 24 hours. After that, the solution was stood, washed twice with 100mL water, and then organic phase and water phase were separated. The organic phase was dried over anhydrous sodium sulfate, and then vacuum distillated to recycle solvent. The resulting residue was recrystallized with isopropanol to obtain 5-cyano-5H-dibenz[b,f]azepine. The yield of the product was 63%, and the purity was 82%.

### Example 10

The feed ratio of carbamazepine : bis(trichloromethyl) carbonate was 1:0.4 (molar ratio), the organic solvent was toluene, and the reaction temperature was 40°C.

A 500mL four-necks flask equipped with a mechanical agitator, constant pressure funnel, reflux condenser and thermometer was charged with 23.60g carbamazepine and 100mL toluene. Opened the agitator, and then a solution consisting of 11.88g bis(trichloromethyl) carbonate in 100mL toluene was added dropwise under vigorous stirring at room temperature. Following the addition, the reaction mixture was maintained at 40°C for 15 hours. After that, the solution was stood, washed twice with 100mL water, and then organic phase and water phase were separated. The organic phase was dried over anhydrous sodium sulfate, and then vacuum distillated to recycle solvent. The resulting residue was recrystallized with isopropanol to obtain 5-cyano-5H-dibenz[b,f]azepine. The yield of the product was 78%, and the purity was 87%.

### Example 11

The feed ratio of carbamazepine : bis(trichloromethyl) carbonate was 1:0.4 (molar ratio), the organic solvent was toluene, and the reaction temperature was 60°C.

A 500mL four-necks flask equipped with a mechanical agitator, constant pressure funnel, reflux condenser and thermometer was charged with 23.60g carbamazepine and 100mL toluene. Opened the agitator, and then a solution consisting of 11.88g bis(trichloromethyl) carbonate in 100mL toluene was added dropwise under vigorous stirring at room temperature. Following the addition, the reaction mixture was maintained at 60°C for 11 hours. After that, the solution was stood, washed twice with 100mL water, and then organic phase and water phase were separated. The organic phase was dried over anhydrous sodium sulfate, and then vacuum distillated to recycle solvent. The resulting residue was recrystallized with isopropanol to obtain 5-cyano-5H-dibenz[b,f]azepine. The yield of the product was 86%, and the purity was 93%.

### Example 12

The feed ratio of carbamazepine : bis(trichloromethyl) carbonate was 1:0.4 (molar ratio), the organic solvent was toluene, and the reaction temperature was 90°C.

A 500mL four-necks flask equipped with a mechanical agitator, constant pressure funnel, reflux condenser and thermometer was charged with 23.60g carbamazepine and 100mL toluene. Opened the agitator, and then a solution consisting of 11.88g bis(trichloromethyl) carbonate in 100mL toluene was added dropwise under vigorous stirring at room temperature. Following the addition, the reaction mixture was maintained at 90°C for 8 hours. After that, the solution was stood, washed twice with 100mL water, and then organic phase and water phase were separated. The organic phase was dried over anhydrous sodium sulfate, and then vacuum distillated to recycle solvent. The resulting residue was recrystallized with isopropanol to obtain 5-cyano-5H-dibenz[b,f]azepine. The yield of the product was 90%, and the purity was 97%.

### Example 13

The feed ratio of carbamazepine : bis(trichloromethyl) carbonate was 1:0.4 (molar ratio), the organic solvent was chloroform, the reaction temperature was the reflux temperature of chloroform.

A 500mL four-necks flask equipped with a mechanical agitator, constant pressure funnel, reflux condenser and thermometer was charged with 23.60g carbamazepine and 100mL toluene. Opened the agitator, and then a solution consisting of 11.88g bis(trichloromethyl) carbonate in 100mL toluene was added dropwise under vigorous stirring at room temperature. Following the addition, the reaction mixture was heated to reflux and then maintained for 6 hours. After that, the solution was stood, washed twice with 100mL water, and then organic phase and water phase were separated. The organic phase was dried over anhydrous sodium sulfate, and then vacuum distillated to recycle solvent. The resulting residue was recrystallized with isopropanol to obtain 5-cyano-5H-dibenz[b,f]azepine. The yield of the product was 97%, and the purity was 98%.

### Example 14

The feed ratio of carbamazepine : bis(trichloromethyl) carbonate was 1:0.4 (molar ratio), the organic solvent was acetone, the reaction temperature was the reflux temperature of acetone.

A 500mL four-necks flask equipped with a mechanical agitator, constant pressure funnel, reflux condenser and thermometer was charged with 23.60g carbamazepine and 100mL toluene. Opened the agitator, a solution consisting of 11.88g bis(trichloromethyl) carbonate in 100mL toluene was added dropwise under vigorous stirring at room temperature. Following the addition, the reaction mixture was heated to reflux and then maintained for 9 hours. After that, the solution was stood, washed twice with 100mL water, and then organic phase and water phase were separated. The organic phase was dried over anhydrous sodium sulfate, and then vacuum distillated to recycle solvent. The resulting residue was recrystallized with isopropanol to obtain 5-cyano-5H-dibenz[b,f]azepine. The yield of the product was 84%, and the purity was 90%.

## Claims

1. A method for preparing 5-cyano-5H-dibenz[b,f]azepine of formula (I), includes the following steps: carbamazepine of formula (II) and bis(trichloromethyl) carbonate of formula (III) are taken as raw materials which react in an organic solvent for 1∼30 hours at 20∼150°C, and reaction solution is post-treated to obtain the 5-cyano-5H-dibenz[b,f]azepine (I);

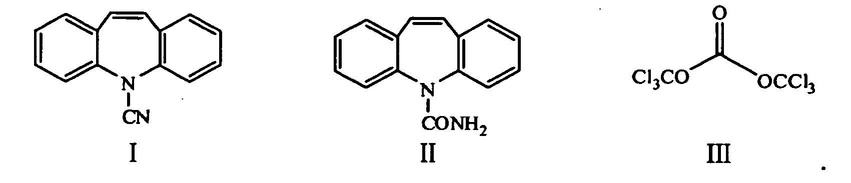

2. The method according to Claim 1, which is **characterized in that** the organic solvent of the present invention is selected from the group consisting of: benzene, toluene, xylene, chlorobenzene, dichloromethane, chloroform, carbon tetrachloride, dichloroethane, tetrahydrofuran, ethyl acetate, acetone, and cyclohexane.

3. The method according to Claim 2, which is **characterized in that** the organic solvent of the present invention is selected from the group consisting of: toluene, xylene, dichloromethane, chloroform, tetrahydrofuran, ethyl acetate, and acetone.

4. The method according to Claim 1, which is **characterized in that** the molar ratio of carbamazepine to bis(trichloromethyl) carbonate is 1.0:0.2∼1.2.

5. The method according to Claim 4, which is **characterized in that** the molar ratio of carbamazepine to bis(trichloromethyl) carbonate is 1.0:0.3∼0.8.

6. The method according to Claim 2, which is **characterized in that** the amount of the organic solvent in mass is 5∼20 times of carbamazepine.

7. The method according to Claim 6, which is **characterized in that** the amount of the organic solvent in mass is 5∼10 times of carbamazepine.

8. The method according to Claim 1, which is **characterized in that** the reaction temperature is 20∼80°C, the reaction time is 4∼15 hours.

9. The method according to one of Claim 1-8, which is **characterized in that** the post-treatment of the present invention may take the following steps: the reaction solution is washed with water, and organic phase and water phase are separated; the organic phase is dried over anhydrous sodium sulfate, and then vacuum distillated to recycle solvent;the resulting residue is recrystallized with isopropanol to obtain 5-cyano-5H-dibenz[b,f]azepine (I).

10. The method according to Claim 1, which is **characterized in that** the method is performed according to the following steps: an organic solvent and carbamazepine (II) are added into the reaction vessel, and then a solution consisting of bis(trichloromethyl) carbonate (III) in the same organic solvent is slowly dropped under vigorous stirring at room temperature; following the addition, the reaction mixture is maintained at 20∼80°C for 4∼15 hours; after the reaction is complete, reaction solution is washed twice with water, and organic phase and water phase are separated; the organic phase is dried over anhydrous sodium sulfate, and then vacuum distillated to recycle solvent; the resulting residue is recrystallized with isopropanol to obtain 5-cyano-5H-dibenz[b,f]azepine (I); The organic solvent is toluene, xylene, dichloromethane, chloroform, tetrahydrofuran, ethyl acetate or acetone; the molar ratio of carbamazepine to bis(trichloromethyl) carbonate is 1.0:0.3∼0.8, and the total amount of the organic solvent is 5∼10 times of the mass of carbamazepine.

## Patentansprüche

1. Verfahren zur Herstellung von 5-Cyano-5H-Dibenz[b,f]azepin der Formel (I), umfassend folgende Stufen: Carbamazepin der Formel (II) und Bis(trichlormethyl) karbonat der Formel (III) werden als Rohstoffe genommen, die in einem organischen Lösungsmittel während 1∼30 Stunden bei 20-150°C reagieren, und die Reaktionslösung wird nachverarbeitet, um 5-Cyano-5H-Dibenz[b,f]azepin (I) zu erhalten;

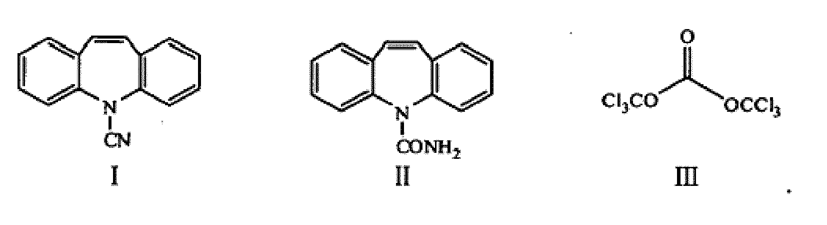

2. Verfahren nach Anspruch 1, **dadurch gekennzeichnet, dass** das organische Lösungsmittel der vorliegenden Erfindung aus der Gruppe gewählt ist, die besteht aus : Benzol, Toluol, Xylol, Chlorbenzol, Dichlormethan, Chloroform, Kohlenstofftetrachlorid, Dichlorethan, Tetrahydrofuran, Essigsäureethylester, Aceton und Cyclohexan.

3. Verfahren nach Anspruch 2, **dadurch gekennzeichnet, dass** das organische Lösungsmittel der vorliegenden Erfindung aus der Gruppe gewählt ist, die besteht aus : Toluol, Xylol, Tetrahydrofuran, Essigsäureethylester und Aceton.

4. Verfahren nach Anspruch 1, **dadurch gekennzeichnet, dass** das molare Verhältnis zwischen Carbamazepin und Bis(trichlormethyl)karbonat 1.0:0.2∼1.2 ist.

5. Verfahren nach Anspruch 1, **dadurch gekennzeichnet, dass** das molare Verhältnis zwischen Carbamazepin und Bis(trichlormethyl)karbonat 1.0:0.3∼0.8 ist.

6. Verfahren nach Anspruch 2, **dadurch gekennzeichnet, dass** die Menge von organischem Lösungsmittel in der Masse 5∼20 Mal größer als diejenige von Carbamazepin ist.

7. Verfahren nach Anspruch 6, **dadurch gekennzeichnet, dass** die Menge von organischem Lösungsmittel in der Masse 5∼10 Mal größer als diejenige von Carbamazepin ist..

8. Verfahren nach Anspruch 1, **dadurch gekennzeichnet, dass** die Reaktionstemperatur 20∼80°C beträgt, die Reaktionszeit 4∼15 Stunden betragt.

9. Verfahren nach einem der Ansprüche 1 bis 8, **dadurch gekennzeichnet, dass** die Nachverarbeitung der vorliegenden Erfindung folgende Schritte annehmen kann : die Reaktionslösung wird mit Wasser gewaschen, und die organische Phase und die wässrige Phase werden getrennt; die organische Phase wird auf wasserfreiem Natriumsulfat getrocknet, und anschließend unter Vakuum destilliert, um das Lösungsmittel wiederzuverwenden ; der sich ergebende Rückstand wird wieder kristallisiert mit Isopropanol, um 5-Cyano-5H-Dbenz[b,f]azepin (I) zu erhalten.

10. Verfahren nach Anspruch 1, **dadurch gekennzeichnet, dass** das Verfahren nach folgenden Stufen durchgeführt wird : ein organisches Lösungsmittel und Carbamazepin (II) werden einem Reaktionsbehälter zugefügt, und anschließend wird eine Lösung, die aus Bis(trichlormethyl)karbonat (III) besteht, langsam tropfweise in dasselbe organische Lösungsmittel unter starker Rührung bei Raumtemperatur zugefügt ; nach der Zufügung ist die Reaktionsmischung vollständig, die Reaktionslösung wird zweimal mit Wasser gewaschen, und die organische Phase und die wässrige Phase werden getrennt ; die organische Phase wird auf wasserfreiem Natriumsulfat getrocknet, und anschließend unter Vakuum destilliert, um das Lösungsmittel wiederzuverwenden ; der sich ergebende Rückstand wird wieder kristallisiert mit Isopropanol, um 5-Cyano-5H-Dbenz[b,f]azepin (I) zu erhalten ; das organische Lösungsmittel ist Toluol, Xylol, Dichlormethan, Chloroform, Tetrahydrofuran, Essigsäureethylester oder Aceton ; das molare Verhältnis zwischen Carbamazepin und Bis(trichlormethyl)karbonat beträgt 1.0:0.3∼0.8, und die Menge von organischem Lösungsmittel in der Masse ist 5∼10 Mal größer als diejenige von Carbamazepin.

## Revendications

1. Procédé de préparation de 5-cyano-5H-dibenz[b,f]azépine de la formule (I), comportant les étapes suivantes : la carbamazépine de la formule (II) et le carbonate de bis(trichlorométyl) de la formule (III) sont pris comme matières brutes qui réagissent dans un solvant organique pendant de 1∼30 heures à de 20 à 150°C, et la solution de réaction est post-traitée pour obtenir de la 5-cyano-5H-dibenz[b,f]azépine (I);

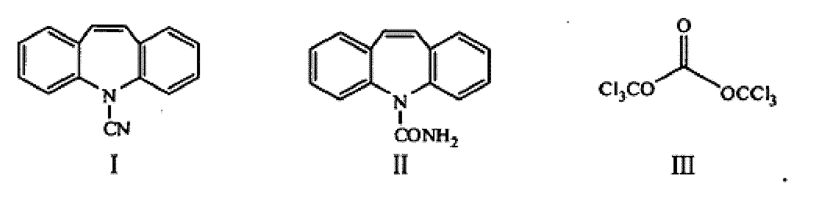

2. Procédé selon la revendication 1, qui est **caractérisé par le fait que** le solvant organique de la présente invention est choisi parmi le groupe composé de : benzène, toluène, xylène, chlorobenzène, dichlorométhane, chloroforme, tétrachlorure de carbone, dichloroéthane, tétrahydrofurane, acétate d'éthyle, acétone et cyclohexane.

3. Procédé selon la revendication 2, qui est **caractérisé par le fait que** le solvant organique de la présente invention est choisi parmi le groupe composé de : toluène, xylène, tétrahydrofurane, acétate d'éthyle et acétone.

4. Procédé selon la revendication 1, qui est **caractérisé par le fait que** le rapport molaire entre la carbamazépine et le carbonate de bis(trichlorométyl) est de 1.0:0.2∼1.2.

5. Procédé selon la revendication 4, qui est **caractérisé par le fait que** le rapport molaire entre la carbamazépine et le carbonate de bis(trichlorométyl) est de 1.0:0.3∼0.8.

6. Procédé selon la revendication 2, qui est **caractérisé par le fait que** la quantité de solvant organique dans la masse est de 5∼20 fois celle de la carbamazépine.

7. Procédé selon la revendication 6, qui est **caractérisé par le fait que** la quantité de solvant organique dans la masse est de 5∼10 fois celle de la carbamazépine.

8. Procédé selon la revendication 1, qui est **caractérisé par le fait que** la température de réaction est de 20∼80°C, le temps de réaction est de 4∼15 heures.

9. Procédé selon l'une des revendications 1 à 8, qui est **caractérisé par le fait que** le post-traitement de la présente invention peut adopter les étapes suivantes : la solution de réaction est lavée avec de l'eau, et la phase organique et la phase aqueuse sont séparées ; la phase organique est séchée sur du sulfate de sodium anhydre, et ensuite distillée sous vide pour recycler le solvant ; le résidu résultant est recristallisé avec de l'isopropanol pour obtenir de la 5-cyano-5H-dibenz[b,f]azépine (I).

10. Procédé selon la revendication 1, qui est **caractérisé par le fait que** le procédé est réalisé selon les étapes suivantes : un solvant organique et de la carbamazépine (II) sont ajoutés dans un récipient de réaction, et ensuite une solution consistant en du carbonate de bis(trichlorométyl) (III) dans le même solvant organique est lentement ajouté goutte à goutte tout en agitant vigoureusement à température ambiante ; après l'ajoute, le mélange de réaction est complet, la solution de réaction est lavée deux fois avec de l'eau, et la phase organique et la phase aqueuse sont séparées ; la phase organique est séchée sur du sulfate de sodium anhydre, et ensuite distillée sous vide pour recycler le solvant ; le résidu résultant est recristallisé avec de l'isopropanol pour obtenir de la 5-cyano-5H-dibenz[b,f]azépine (I) ; le solvant organique est toluène, xylène, dichlorométhane, chloroforme, tétrahydrofurane, acétate d'éthyle ou acétone ; le rapport molaire entre la carbamazépine et le carbonate de bis(trichlorométyl) est de 1.0:0.3∼0.8, et la quantité de solvant organique dans la masse est de 5∼10 fois celle de la carbamazépine.
